(19) Europäisches Patentamt
European Patent Office
Office européen des brevets

(11) **EP 4 686 447 A1**

(12) **EUROPEAN PATENT APPLICATION**

(43) Date of publication:
**04.02.2026 Bulletin 2026/06**

(21) Application number: **25192243.1**

(22) Date of filing: **28.07.2025**

(51) International Patent Classification (IPC):
**A61B 5/1455** (2006.01)   **A61B 5/00** (2006.01)

(52) Cooperative Patent Classification (CPC):
**A61B 5/14552; A61B 5/6826; A61B 5/7221;**
A61B 2562/0238

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **01.08.2024 US 202463678217 P**

(71) Applicant: **Drägerwerk AG & Co. KGaA
23558 Lübeck (DE)**

(72) Inventor: **MASHIMO, Minoru
Windham, 03087 (US)**

(74) Representative: **Haseltine Lake Kempner LLP
One Portwall Square
Portwall Lane
Bristol BS1 6BH (GB)**

(54) **PULSE OXIMETRY BY REMOVING THE EFFECTS OF TISSUE SCATTERING**

(57)   A pulse oximeter and method of calculating blood oxygen saturation that enables increased accuracy when signal levels and/or quality are low, as well as across a range of skin tones. Increased accuracy is enabled by detecting red and IR LED signals using both transmissive and reflective light detectors. In addition, several criteria are used to evaluate whether the detected transmissive or reflective signal provides a more accurate blood oxygen saturation value.

FIG. 1

**Description**

**CROSS REFERENCE TO RELATED APPLICATIONS**

The present application claims priority to and the benefit of U.S. Prov. Pat. App. Ser. No. 63/678,217, which was filed on August 1, 2024, for all purposes, including the right of priority, which application is hereby incorporated herein by reference in its entirety and to the extent that is not inconsistent with the present disclosure.

**BACKGROUND**

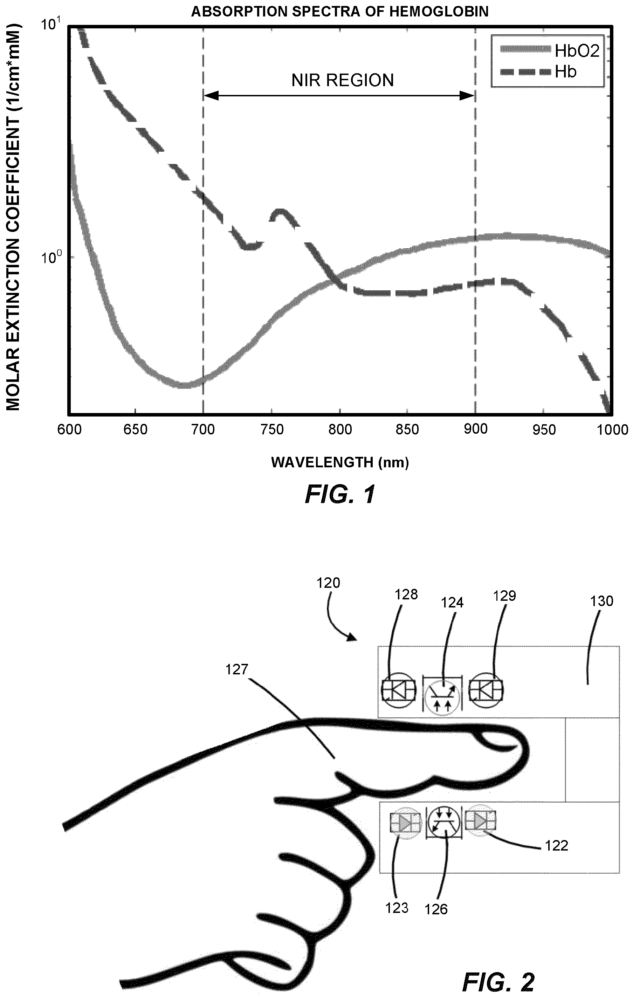

Pulse oximetry is the non-invasive measurement of the oxygen saturation (SpO2). Oxygen saturation is defined as the measurement of the amount of oxygen dissolved in blood, based on the detection of Hemoglobin (HbO2) and Deoxyhemoglobin (Hb). The bloodstream is affected by the concentration of HbO2 and Hb, and their absorption coefficients are measured (using a photodetector) using two wavelengths 660 nm (red light spectra) and 940 nm (infrared light spectra). Hb and HbO2 absorb different wavelengths. Hb has a higher absorption at 660 nm and HbO2 has a higher absorption at 940 nm (Figure 1).

Existing methods of measuring oxygen saturation use Lambert-Beer's Law (Equation 1) and/or its derivative.

$$I = I_o \cdot e^{-b_{eff} \, c \, l} \tag{1}$$

Where:

$b_{eff}$   - is the extinction coefficient
$c$      - is the concentration of the absorption medium
$l$      - is the path length of light

The photodetector is also adapted to detect a pulse and measure a pulse rate. For most applications where a patient's condition is stable or healthy, logarithm calculations employing pulsatile (AC) and non-pulsative (DC) signals to derive maximum dynamic range. A perfusion index ("PI") may also be used as part of the AC/DC method an indicator of SpO2 signal quality. PI is calculated by dividing a pulsatile signal (AC) by a non-pulsative signal (DC) and multiplying the result by 100 to provide the result as a percentage, which typically ranges from 0.02% to 20%.

Calculating SpO2 using a conventional AC/DC method relies on assumptions that may contribute to inaccurate SpO2 readings. First, it assumes that the LED light spectrum is consistent for all LEDs manufactured for a given wavelength. There are often variations in the LED light spectrum among LEDs manufactured for a given wavelength. Second, it assumes that the distance between the light source and the detector is consistent. It is not uncommon for that distance to vary due to motion of the patient's finger (or ear) relative to the pulse oximeter device. Third, it assumes that light scatter by tissue is negligible. When a patient's tissue is subjected to intense light, it can absorb or reflect incident light. Fourth, it assumes that blood flow is always measured between its peaks and valleys. This is challenging to achieve in practice and limits the ability to obtain accurate SpO2 readings when a patient's pulse is irregular or erratic. Fifth, it does not account for differences in the light absorption/scattering based on different skin tones. Therefore, there is a pulse oximeter system and method that provides more accurate SpO2 measurements across a wide range of skin tones and in situations in which the patient's pulse is irregular or erratic.

**SUMMARY**

This summary is provided to introduce a selection of concepts in a simplified form that are further described in more detail below. This summary is not intended to identify key features or essential features of the invention, nor is it intended to be used to limit the scope of the invention.

Several aspects of the systems and methods of the invention are outlined below.

Aspect 1: A pulse oximeter comprising:

a plurality of light emitters comprising a first transmissive light emitter adapted to emit light at a first wavelength and a second transmissive light emitter adapted to emit light at a second wavelength, the second wavelength being different from the first wavelength;
a transmissive detector adapted to detect light at the first and second wavelength, convert light detected at the first wavelength into a first transmissive signal and to convert light detected at the second wavelength into a second transmissive signal;

a reflective detector adapted to detect light at the first and second wavelength, convert light detected at the first wavelength into a first reflective signal and to convert light detected at the second wavelength into a second reflective signal; and

a housing adapted to provide a tissue opening located between a first half and a second half, the tissue opening being adapted to receive human tissue therein;

wherein the transmissive detector is located in the first half of the housing and the first transmissive light emitter, the second transmissive light emitter, and the reflective detector are located in the second half of the housing; and

wherein the first transmissive light emitter, the second transmissive light emitter are oriented to direct light at the transmissive detector.

Aspect 2: The pulse oximeter of Aspect 1, wherein the plurality of light emitters further comprises a first reflective light emitter adapted to emit light at the first wavelength and a second reflective light emitter adapted to emit light at the second wavelength, the first reflective light emitter and the second reflective light emitter being located in the first half of the housing.

Aspect 3: The pulse oximeter of any of Aspects 1-2, wherein the human tissue being at least one selected from the group of a finger, a toe, an earlobe, a nose, or a forehead.

Aspect 4: The pulse oximeter of any of Aspects 1-3, wherein the first wavelength is infrared.

Aspect 5: The pulse oximeter of any of Aspects 1-5, wherein the second wavelength is red.

Aspect 6: The pulse oximeter of any of Aspects 1-7, further comprising at least one processor adapted to receive a pulse signal and determine a blood oxygen saturation measurement based on the pulse signal and at least one of (a) the first and second transmissive signals and (b) the first and second reflective signals.

Aspect 7: The pulse oximeter of Aspect 6, further comprising a differentiator in communication with the log amplifier.

Aspect 8: A pulse oximeter comprising:

a housing adapted to provide a tissue opening located between a first half and a second half, the tissue opening being adapted to receive human tissue therein, the first half opposing the second half;

a plurality of light emitters comprising a first transmissive light emitter adapted to emit light at a first wavelength and a second transmissive light emitter adapted to emit light at a second wavelength, the second wavelength being different from the first wavelength, the plurality of light emitters being located in the first half of the housing;

a transmissive detector adapted to detect light at the first and second wavelength, convert light detected at the first wavelength into a first transmissive signal and to convert light detected at the second wavelength into a second transmissive signal, the transmissive detector being located in the second half of the housing, wherein the first transmissive light emitter and the second transmissive light emitter are oriented to direct light at the transmissive detector;

a reflective detector adapted to detect light at the first and second wavelength, convert light detected at the first wavelength into a first reflective signal and to convert light detected at the second wavelength into a second reflective signal, the reflective detector being located in the first half of the housing; and

a processor configured to:

determine whether each of a plurality of criteria is met, the plurality of criteria comprising a set of error criteria, a set of transmissive criteria, and a set of reflective criteria;

if the set of error criteria is determined to be met and the set of transmissive criteria is determined to be met, calculating and displaying a blood oxygen saturation value based on the first and second transmissive signals;

if the set of error criteria is determined to be met, the set of transmissive criteria is determined not to be met, and the set of reflective criteria determined to be met, calculating and displaying the blood oxygen saturation value based on the first and second reflective signals; and

if the set of error criteria is determined to be met, displaying an error condition.

Aspect 9: The pulse oximeter of Aspect 8, wherein the set of transmissive criteria comprises the transmissive signal having a perfusion index (PI) that is equal to or greater than predetermined PI value.

Aspect 10: The pulse oximeter of any of Aspects 8-9, wherein the set of transmissive criteria comprises an amplifier being at less than maximum gain when the transmissive signal is set at 50% and below a saturation level for a log amplifier.

Aspect 11: The pulse oximeter of any of Aspects 8-10, wherein the set of reflective criteria comprises the reflective signal having a perfusion index that is greater than a perfusion index of the transmissive signal.

Aspect 12: The pulse oximeter of any of Aspects 8-11, wherein the set of reflective criteria comprises a first blood oxygen saturation value calculated based on the reflective signal not being equal to a second blood oxygen saturation value being calculated based on the transmissive signal.

Aspect 13: The pulse oximeter of any of Aspects 8-12, wherein the plurality of light emitters further comprises a first reflective light emitter adapted to emit light at the first wavelength and a second reflective light emitter adapted to emit light at the second wavelength, the first reflective light emitter and the second reflective light emitter being located in the first half of the housing.

Aspect 14: The pulse oximeter of any of Aspects 8-13, wherein the first wavelength is infrared.

Aspects 15: A method of measuring blood oxygen saturation in a tissue, the method comprising:

determining whether each of a plurality of criteria is met, the plurality of criteria comprising a set of error criteria, a set of transmissive criteria, and a set of reflective criteria;

if the set of error criteria is determined to be met and the set of transmissive criteria is determined to be met, calculating and displaying a blood oxygen saturation value for the tissue a based on a transmissive signal generated by a transmissive detector positioned on an opposite side of the tissue from a first transmissive light emitter adapted to emit light at a wavelength a range of 610nm to 710 nm and a second transmissive light emitter adapter adapted to emit light at a wavelength a range of 890nm to 990nm;

if the set of error criteria is determined to be met, the set of transmissive criteria is determined not to be met, and the set of reflective criteria determined to be met, calculating and displaying the blood oxygen saturation value based on a reflective signal generated by a reflective detector positioned on a same side of the tissue from the first transmissive light emitter and the second transmissive light emitter; and

if the set of error criteria is determined to be met, displaying an error condition.

## BRIEF DESCRIPTION OF THE DRAWINGS

The present invention will hereinafter be described in conjunction with the appended drawing figures wherein like numerals denote like elements.

Figure 1 is a graph showing absorption of HbO2 and Hb across a range of wavelengths of light.

Figure 2 is a schematic diagram showing an exemplary probe for a pulse oximeter attached to a finger.

Figure 3 is a block diagram showing exemplary signal processing hardware for the pulse oximeter shown in Figure 2.

Figure 4 is an exemplary method of operating the pulse oximeter shown in Figures 2 and 3.

## DETAILED DESCRIPTION

The ensuing detailed description provides preferred exemplary embodiments only, and is not intended to limit the scope, applicability, or configuration of the invention. Rather, the ensuing detailed description of the preferred exemplary embodiments will provide those skilled in the art with an enabling description for implementing the preferred exemplary embodiments of the invention. It being understood that various changes may be made in the function and arrangement of elements without departing from the spirit and scope of the invention, as set forth in the appended claims.

To aid in describing the invention, directional terms are used in the specification and claims to describe portions of the present invention (e.g., upper, lower, left, right, etc.). These directional definitions are merely intended to assist in describing and claiming the invention and are not intended to limit the invention in any way. In addition, reference numerals that are introduced in the specification in association with a drawing figure may be repeated in one or more subsequent figures without additional description in the specification in order to provide context for other features.

When a patient's tissue is subjected to intense light, it both absorbs and reflects incident light. When patient motion is introduced between an LED emitter and a photodetector, it becomes more difficult to overcome the irregularities in pulse rate and pulse amplitudes using AC and DC values. Light scattering modifies the curve index and/or coefficients to the polynomial for calculating saturated oxygen. The following steps may offset these effects.

The intensity of LED emitters may be varied, instead of simply being used in 'on' and 'off', states which is described using Rayleigh's scattering equation (Equation 2):

$$I(\theta) = I_0 \left(\frac{R^6}{l^2}\right)(1 + \cos^2\theta)(\lambda^{-4}) \tag{2}$$

Where:

$I(\theta)$     Intensity of scattered light at an angle

$I_0$     Intensity of incident light

$R$     Size of scattering particles

$l$     Distance between particle and observer

$\theta$     Angle between incident light and scattered light

$\lambda$     Wavelength of incident light

This equation dictates that intensity of scattered light is inversely proportional to the wavelength by a power of four. Between red (680nm) and infrared (960nm) LED light, any molecule in the light's path can scatter and attenuate up to 4-times (3.97x), as expressed by Equation 3:

$$b_{RedScatter} = 3.97 * b_{IRScatter} \qquad (3)$$

Using different ambient light levels, the effect of scattering can be assessed by comparing ratios. Alternating intensities of red and IR are used to calculate ($I_0$ - $I$) extinction coefficients. Pulse rates are computed using the resulting coefficient value with static effects of light scatter removed. This is computed using Lambert-Beer's Law (Equation 4):

$$I = I_0 * e^{-b_{eff}cl} \qquad (4)$$

Where:

$I$     Resultant intensity

$I_0$     Incident intensity

$b_{eff}$     Extinction coefficient

$c$     Concentration of absorbing medium

$l$     Length light travels in medium

Extinction Coefficient(s) for hemoglobin (Equation 5):

$$b_{eff} = S * b_{HbO2} + (1 - S)b_{Hb} \qquad (5)$$

Where:

$b_{HbO2}$     Extinction coefficient for oxygenated hemoglobin
$b_{Hb}$     Extinction coefficient for deoxygenated hemoglobin

Using both red and IR LEDs arranged side-by-side and photodetectors in 'transmissive' mode allows concentration (c) and length (I) to be factored out (Equation 6):

$$\frac{ln\frac{I_{max}}{I_{min}}|_{Red}}{ln\frac{I_{max}}{I_{min}}|_{IR}} = R = \frac{[A*S+B*(1-S)]}{[C*S+D*(1-S)]} \qquad (6)$$

Where:

A, B     $b_{eff}$ of Red LED oxygenated and de-oxygenated hemoglobin
C, D     $b_{eff}$ of IR LED oxygenated and de-oxygenated hemoglobin

Using Red and IR LEDs coefficients provided by Nellcor, since extinction coefficients are tested for LED spectrum, a reasonable assumption can be made that when LED light hits tissues other than hemoglobin, light attenuation is caused by other types of absorption including scatter (Equation 7):

$$R = \frac{[A*S+B*(1-S)]+b_{RedScatter}}{[C*S+D*(1-S)]+b_{IRScatter}} = \frac{[A*S+B*(1-S)]+b_{RedScatter}}{[C*S+D*(1-S)]+(3.97*b_{RedScatter})} \tag{7}$$

Saturation varies by the following equation (Equation 8):

$$S = \frac{B-D*R+(3.97*b_{RedScatter}*R)}{R*(C-D)+(B-A)+b_{RedScatter}} \tag{8}$$

Two different sensors or LED locations may be used - one for normal transmissive light and the other for reflective or scattered light detection. Results of both logarithmic computations derive extinction coefficients by factoring out the concentration value. Transmissive and reflective pulsating signals are detecting oxygenated blood. Accordingly, if both transmissive and reflective signals are accurate, the ratios should not be different. If the ratios are different, then the ratio that has the least $b_{Scatter}$ effect should be used. In one embodiment, the preferred saturated oxygen value (i.e. transmissive or reflective) is chosen by the better ratio with minimal scatter given an approximation for $R_{trans}$ versus $R_{reflect}$ (Equation 9):

$$b_{RedScatter} \approx \frac{A-R*C}{1-3.97*R} \tag{9}$$

Choose the ratio given the lesser $b_{RedScatter}$ (this can be refactored into equation 10):

$$\frac{A-R_{trans}*C}{1-3.97*R_{trans}} \lessgtr \frac{A-R_{reflect}*C}{1-3.97*R_{reflect}} \tag{10}$$

As will be explained herein, in order to provide more accurate SpO2 readings across a range of skin tones, it is desirable to ensure that the pulse oximeter probe is oriented with the reflective photodetector on the palm side of a finger (i.e., opposite the nail), where less melanin is present. However, it may be possible in some embodiments to place the sensor on a fingernail (if there is no fingernail polish) rather than the palm-side of the finger. This would mitigate for placement of the LED and photodiode sensors close to the end of the fingertip rather than in the middle of the finger. Such a change would, in turn, implicate the use of a soft light-blocking material, such as a dark foam, at the fingertip to create a comfortable finger "stop" in the probe.

Referring to Figure 2, an exemplary probe 120 for a pulse oximeter is shown. The probe 120 is shown on a finger 127 of a human patient. It could, alternatively, be placed on an ear lobe, toe, nose or forehead of a patient. The probe 120 includes a housing 130, red LED 122 designed to emit light at 660 nm, and an infrared LED 123 designed to emit light at 940mn (also referred to herein as transmissive light emitters).

The probe 120 includes a transmissive photodetector 124 that is positioned on the opposite side of the finger 127 from the LEDs 122, 123 and a reflective photodetector 126 positioned on the same side of the finger 127 as the LEDs 122, 123. The transmissive photodetector 124 detects light from the LEDs 122, 123 that has passed through the tissue of the finger 127. The reflective photodetector 126 detects light from the LEDs 122, 123 that has been reflected by the tissue of the finger 127.

In the illustrated embodiment, a second set of LEDs (a red LED 128 and an IR LED 129) is located on the opposite side of the finger 127 from the LEDs 122, 123 and the reflective photodetector 126. The second set of LEDs 128, 129 (also referred to herein as reflective light emitters) can serve several beneficial purposes, including verifying the orientation of the probe 120 on the finger 127. In order to increase accuracy of the probe 120 across a wide range of skin tones, it is desirable for the LEDs 122, 123 to be on the palm side of the finger 127 (i.e., opposite the fingernail), where there is less melanin in the skin.

A light barrier (not shown) could be provided to reduce the amount of ambient light (which may include both red and IR wavelengths of light) that reaches the transmissive and reflective photodetectors 124, 126. Such a barrier may be, for example, a light blocking shroud including a black "bandage". Such a barrier may prevent or inhibit light leakage through the sensing device rather than through the tissue. This may be particularly advantageous in disposable sensing devices.

Due to operating conditions and/or manufacturing, there may be some variation in the actual wavelength emitted by each LED 122, 123, 128, 129. The actual wavelength of each red LED 122, 128 is preferably between 610nm to 710nm and, more preferably, between 650nm and 670nm. Similarly, the actual wavelength of each IR LED 123, 129 is preferably between 890nm to 990nm and, more preferably, between 930nm and 950nm. Currently, LEDs are the only commercially available light sources that emit a light spectrum that is sufficiently narrow for pulse oximeter applications. If other narrow-spectrum light-emitting devices become available, they could be used instead of LEDs.

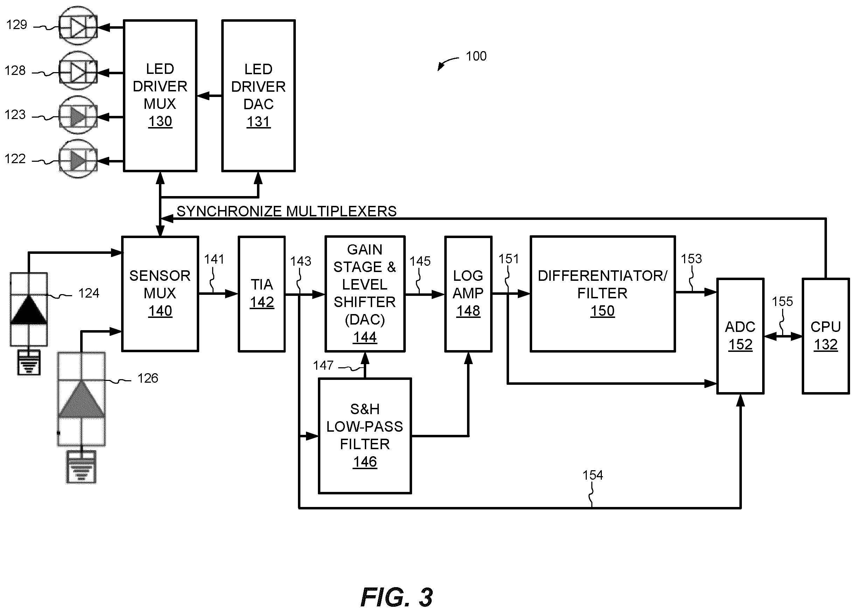

Referring to Figure 3, exemplary signal processing hardware provided as part of an exemplary pulse oximeter 100 is shown. An LED multiplexer 130 and an LED driver 131 provide power to the LEDs 122, 123, 128, 129 and control the intensity and timing of their activation. A sensor multiplexer 140 provides power to the transmissive and reflective photodetectors 124, 126. A processor 132 controls and synchronizes operation of the LEDs 122, 123, 128, 129 and photodetectors 124, 126.

A transimpedance amplifier 142 converts current signals 141 from the photodetectors 124, 126 to a voltage signal 141 and provides gain, resulting in an amplified signal 143 (also referred to herein as an X-TIA signal). A level shifter 144 then removes DC (including noise) from the amplified signal 143, to prevent saturation of the log amplifier 148 and resulting in a reduced noise signal 145. A sample and hold ("S&H") 146 is used to adjust the level shifter 144 (via signal 147) and is driven by a low pass filter. The log amplifier 148 converts the reduced noise signal 145 into its logarithmic value, resulting in a log signal 151. A differentiator 150 (also called a first order high-pass filter) then provides the slope of the log signal 149, resulting in a differentiator signal 153. The differentiator signal 153 can be used to differentiate blood flow from other motion artifacts and/or noise in the log signal 151. Both the log signal 151 and the differentiator signal 153 are then passed to an analog-to-digital convertor 152, which provides a digital signal 155 to the processor 132.

As noted above, a processor 132 controls and synchronizes operation of the LEDs 122, 123, 128, 129 and photodetectors 124, 126. The LEDs may be turned on and off in the following sequence: Red LED 122 on, then both LEDs 122, 123 off, IR LED 123 on and both LEDs 122, 123 off. When on, the LEDs may also be set at a lower intensity for more translucent skin/tissue. The applicable photodetector 124, 126 detects the light emitted from the LED, along with any ambient light. In order to more accurately calculate SpO2, it is desirable to measure ambient light (i.e., with all LEDs off) received by the applicable photodetector 124, 126 and "subtract" the ambient light portion of the signal when measuring a signal from the red LED 122 or IR LED 123. Changes in ambient light may also be used to detect or assess artifacts introduced by motion, as such changes not only effect AC & DC, but also the slope of the signal detected by the differentiator 150.

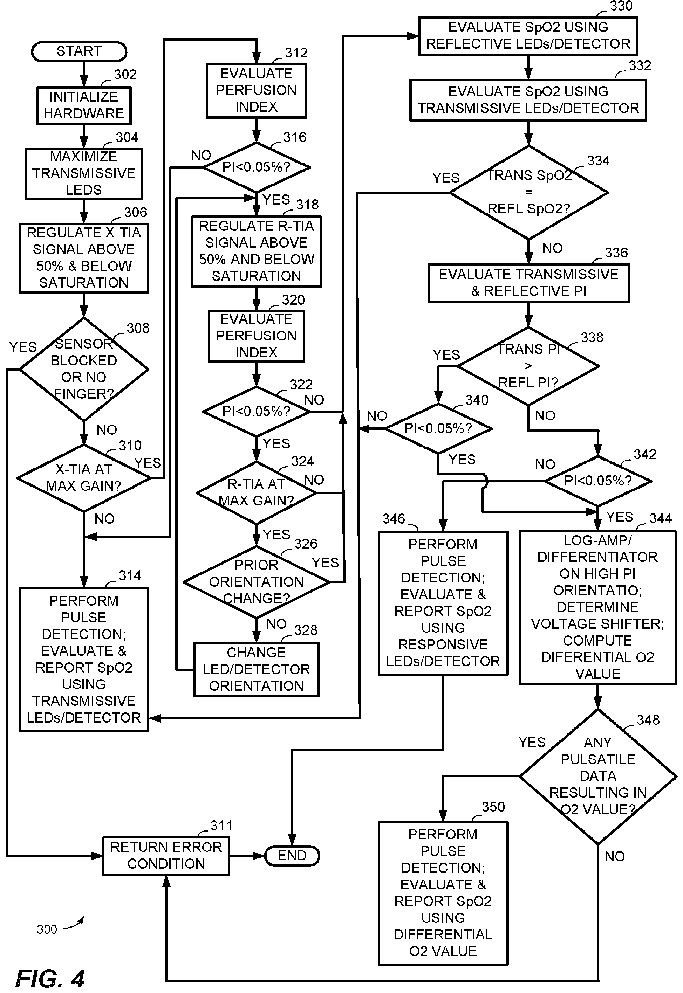

Figure 4 illustrates an exemplary method 300 of calculating SpO2 using the system 200 of Figure 2. The process begins by initializing hardware (step 302) and sequentially activating the transmissive LED emitters (LED 122, 123) to maximum intensity (step 304) and measuring light through the transmissive photodetector 124. The amplified transmissive ("X-TIA") signal is then regulated by the level shifter 144 and the sample and hold circuit 146 to be above 50% and below saturation for the log amplifier 148 (step 306).

Next, the system determines if the transmittance photodetector 126 is blocked or no finger is present (step 308). If 0% transmittance (opaque) is detected, then the transmittance photodetector 124 is determined to be blocked or fully misaligned. If there is close to 100% transmittance (transparent) then there is no finger 127 present. (Note that a no-finger-present condition may also result from LED regulation where response to increasing and decreasing LED regulation cannot be detected by the photodiodes. This regulation is particularly sensitive to light leakage through the sensing device and between the LED and the photodiodes rather than through the tissue.) If either error is detected, the process stops, and the user is notified of an error condition (step 311). Optionally, the error condition step 311 could provide a message or indicator that specifies whether there is a blockage / misalignment or no finger 127 present.

After step 308, the X-TIA signal is measured to determine if it is at or below maximum gain for the log amplifier 148 (step 310). If the log amplifier 148 or the level shifter 144 is already "pegged" (at maximum gain), the X-TIA signal is determined to be at maximum gain. If X-TIA gain is below the maximum value, then a pulse detection is performed and SpO2 is evaluated and reported using the X-TIA signal (step 314).

If X-TIA signal gain is at maximum, the system 100 concludes there is a usable signal that warrants additional analysis. First, a perfusion index is calculated for the X-TIA signal (step 312). If the PI is equal to or greater than 0.05% (step 316), then a pulse detection is performed and SpO2 is evaluated and reported using the X-TIA signal (step 314). In this implementation, PI may be evaluated using signals from both red and IR LEDs 122, 123. It is common in the prior art to only evaluate PI based on the red LED 122. However, improved PI accuracy across a range of different skin-tones can be realized by calculating PI based on both signals.

If the PI is less than 0.05% (step 316), then the system 100 determines that evaluating SpO2 saturation using a reflective amplified reflective ("R-TIA") signal value is warranted. Then a series of orientation tests is performed to determine whether a change in orientation of the probe 120 is needed (step 328) of further evaluation analysis is to be performed (starting with step 330). PI is calculated (step 320).

If PI is equal to or greater than a predetermined value (step 322), e.g., 0.05% in this embodiment, then the patient has a strong enough pulse for the transmissive SpO2 saturation value to be meaningful and the next orientation test is performed. If PI is less than the predetermined value, then a message or alert is generated indicating that the orientation of the probe 120 needs to be changed (step 328) and the process returns to step 318.

The next orientation test is to determine if the R-TIA signal at maximum gain (step 324), if so, the user is prompted to change the LED/detector orientation (step 328) and return to step 318. Optionally, the user may be prompted again to change the LED/detector orientation (step 328) if the R-TIA signal is determined to be at maximum gain (step 324) after the first change in orientation. If the R-TIA signal is not at maximum gain, then analysis continues to step 330.

The last orientation test is to determine whether there has been a previous orientation change (step 326). If so, then the process proceeds to step 330. If not, then process proceeds to step 328.

An optional modification to the repeated performance of step 318 (after returning to step 318 from step 328) can be provided if reflective red LED 128 and IR LED 129 are present. When step 318 is repeated, the R-TIA signal could be provided by sequentially activating the reflective LEDs 128, 129 and measuring the signal from the transmissive photodetector 124.

If the orientation tests determine that additional analysis is to proceed, then both an R-TIA SpO2 value (step 330) and an X-TIA SpO2 value (step 332) are calculated. The R-TIA and X-TIA SpO2 values are then compared (step 334). If the values are substantially equal, then SpO2 is reported based on the transmissive SpO2 saturation value (step 314). For purposes of this calculation "substantially equal" may mean that the SpO2 values are within 5% of one another or, more preferably, within 3% of one another. If the values are not substantially equal, then the PI values for the transmissive and reflective saturation values are evaluated (step 336) and compared (step 338). The saturation value with the lower PI is discarded. If the highest PI value is above the predetermined value (e.g., 0.05%) (step 340 or step 342, depending upon which PI value was determined to be higher), then that value is reported as the SpO2 value (step 314 if X-TIA and step 346 if R-TIA). If the highest PI value is determined to not be above the predetermined value (e.g., 0.05%), then the process proceeds to step 344. Optionally, if either the transmissive or reflective SpO2 value is reported as being above 100%, that value could be discarded.

In step 344, gain is adjusted using 144 gain-stage to increase the LED signal (both red and IR) such that the smaller AC value (undulations) is amplified. Due to the fact that amplifiers multiply along the zero voltage axis, an offset is needed to amplify the smaller AC signal (above the zero voltage axis). This is accomplished using the sample-hold/low-pass filter 146, which is then fed back to the level-shifter in the gain-stage 144, essentially removing the effects of high DC. The combined signal is then fed into the log amplifier 148 and the differentiator / high-pass filter (150). Software is used to control operation of the level shifter 144, sample and hold filter 146, the log amplifier 148, and the differentiator / filter 150 to determine if there are any undulations in differentiator signal 153 (step 348). If no undulation is detected in the differentiator signal 153 in step 348, it is determined that the system cannot compute SpO2 and an error is returned (step 310). If undulation is detected, an SpO2 value is returned based on the differential signal 153 (step 350).

In the method 300 as described above, the perfusion index is measured against a threshold of 0.05%. In some embodiments, a low perfusion index of less than 0.02% may cause a numeric under-flow or floating point precision underflow. This may affect the provision of pulse oximetry measurements in those embodiments.

The method described above enables the pulse oximeter to compute more accurate blood oxygen saturation calculations, particularly in instances where signal levels and/or quality are low. The method calculates and reports a blood oxygen saturation value based on the transmissive signal if a certain set of transmissive signal criteria are met (e.g., steps 310 and 316). If the transmissive signal criteria are not met, then additional analysis is performed to determine whether to base the reported blood oxygen saturation value on the reflective signal or the transmissive signal. This additional analysis includes a set of reflective signal criteria (e.g., steps 334, 338, 342). If the reflective criteria are met, then the blood oxygen saturation value to be reported is calculated based on the reflective signal.

While the principles of the invention have been described above in connection with preferred embodiments, it is to be clearly understood that this description is made only by way of example and not as a limitation of the scope of the invention.

**Claims**

1. A pulse oximeter comprising:

a plurality of light emitters comprising a first transmissive light emitter adapted to emit light at a first wavelength and a second transmissive light emitter adapted to emit light at a second wavelength, the second wavelength being different from the first wavelength;
a transmissive detector adapted to detect light at the first and second wavelength, convert light detected at the first wavelength into a first transmissive signal and to convert light detected at the second wavelength into a second transmissive signal;
a reflective detector adapted to detect light at the first and second wavelength, convert light detected at the first wavelength into a first reflective signal and to convert light detected at the second wavelength into a second reflective signal; and
a housing adapted to provide a tissue opening located between a first half and a second half, the tissue opening being adapted to receive human tissue therein;
wherein the transmissive detector is located in the first half of the housing and the first transmissive light emitter, the second transmissive light emitter, and the reflective detector are located in the second half of the housing; and
wherein the first transmissive light emitter, the second transmissive light emitter are oriented to direct light at the transmissive detector.

2. The pulse oximeter of claim 1, wherein the plurality of light emitters further comprises a first reflective light emitter adapted to emit light at the first wavelength and a second reflective light emitter adapted to emit light at the second wavelength, the first reflective light emitter and the second reflective light emitter being located in the first half of the housing.

3. The pulse oximeter of claim 1-2, wherein the human tissue being at least one selected from the group of a finger, a toe, an earlobe, a nose, or a forehead.

4. The pulse oximeter of claim 1-3, wherein the first wavelength is infrared.

5. The pulse oximeter of claim 1-4, wherein the second wavelength is red.

6. The pulse oximeter of claim 1-5, further comprising at least one processor adapted to receive a pulse signal and determine a blood oxygen saturation measurement based on the pulse signal and at least one of (a) the first and second transmissive signals and (b) the first and second reflective signals.

7. The pulse oximeter of claim 6, further comprising a differentiator in communication with the log amplifier.

8. A pulse oximeter comprising:

a housing adapted to provide a tissue opening located between a first half and a second half, the tissue opening being adapted to receive human tissue therein, the first half opposing the second half;
a plurality of light emitters comprising a first transmissive light emitter adapted to emit light at a first wavelength and a second transmissive light emitter adapted to emit light at a second wavelength, the second wavelength being different from the first wavelength, the plurality of light emitters being located in the first half of the housing;
a transmissive detector adapted to detect light at the first and second wavelength, convert light detected at the first wavelength into a first transmissive signal and to convert light detected at the second wavelength into a second transmissive signal, the transmissive detector being located in the second half of the housing, wherein the first transmissive light emitter and the second transmissive light emitter are oriented to direct light at the transmissive detector;
a reflective detector adapted to detect light at the first and second wavelength, convert light detected at the first wavelength into a first reflective signal and to convert light detected at the second wavelength into a second reflective signal, the reflective detector being located in the first half of the housing; and
a processor configured to:

determine whether each of a plurality of criteria is met, the plurality of criteria comprising a set of error criteria, a set of transmissive criteria, and a set of reflective criteria;
if the set of error criteria is determined to be met and the set of transmissive criteria is determined to be met, calculating and displaying a blood oxygen saturation value based on the first and second transmissive signals;
if the set of error criteria is determined to be met, the set of transmissive criteria is determined not to be met, and the set of reflective criteria determined to be met, calculating and displaying the blood oxygen saturation value based on the first and second reflective signals; and
if the set of error criteria is determined to be met, displaying an error condition.

9. The pulse oximeter of claim 8, wherein the set of transmissive criteria comprises the transmissive signal having a perfusion index (PI) that is equal to or greater than predetermined PI value.

10. The pulse oximeter of claim 8-9, wherein the set of transmissive criteria comprises an amplifier being at less than maximum gain when the transmissive signal is set at 50% and below a saturation level for a log amplifier.

11. The pulse oximeter of claim 8-10, wherein the set of reflective criteria comprises the reflective signal having a perfusion index that is greater than a perfusion index of the transmissive signal.

12. The pulse oximeter of claim 8-11, wherein the set of reflective criteria comprises a first blood oxygen saturation value calculated based on the reflective signal not being equal to a second blood oxygen saturation value being calculated based on the transmissive signal.

13. The pulse oximeter of claim 8-12, wherein the plurality of light emitters further comprises a first reflective light emitter adapted to emit light at the first wavelength and a second reflective light emitter adapted to emit light at the second wavelength, the first reflective light emitter and the second reflective light emitter being located in the first half of the housing.

14. The pulse oximeter of claim 8-13, wherein the first wavelength is infrared and the second wavelength is red.

15. A method of measuring blood oxygen saturation in a tissue, the method comprising:

determining whether each of a plurality of criteria is met, the plurality of criteria comprising a set of error criteria, a set of transmissive criteria, and a set of reflective criteria;
if the set of error criteria is determined to be met and the set of transmissive criteria is determined to be met, calculating and displaying a blood oxygen saturation value for the tissue a based on a transmissive signal generated by a transmissive detector positioned on an opposite side of the tissue from a first transmissive light emitter adapted to emit light at a wavelength a range of 610nm to 710 nm and a second transmissive light emitter adapter adapted to emit light at a wavelength a range of 890nm to 990nm;
if the set of error criteria is determined to be met, the set of transmissive criteria is determined not to be met, and the set of reflective criteria determined to be met, calculating and displaying the blood oxygen saturation value based on a reflective signal generated by a reflective detector positioned on a same side of the tissue from the first transmissive light emitter and the second transmissive light emitter; and
if the set of error criteria is determined to be met, displaying an error condition.

ABSORPTION SPECTRA OF HEMOGLOBIN

FIG. 1

FIG. 2

FIG. 3

FIG. 4

Europäisches
Patentamt

European
Patent Office

Office européen
des brevets

# EUROPEAN SEARCH REPORT

**Application Number**

**EP 25 19 2243**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 8 761 850 B2 (MASIMO CORP [US]) 24 June 2014 (2014-06-24) | 1-6 | INV. A61B5/1455 |
| Y | * column 4, line 38 - column 5, line 57; claim 1; figures 1, 2, 3A, 3B * | 7 | A61B5/00 |
| X | US 2013/035562 A1 (BESKO DAVID P [US]) 7 February 2013 (2013-02-07) * paragraphs [0022] - [0038], [0044]; claims 1-20; figures 2A, 2B, 3, 4, 7 * | 8,9, 13-15 | |
| Y | EP 0 352 923 A1 (BAXTER INT [US]) 31 January 1990 (1990-01-31) * page 5, line 36 - page 6, line 1; figure 1 * | 7 | |
| A | Nellcor: "OxiMax N-560 - Pulse Oximeter Operator's Manual", , 24 January 2006 (2006-01-24), XP093329575, Retrieved from the Internet: URL:https://rtmedical.org/wp-content/uploads/2019/11/Nellcor-N560-user-manual.pdf?utm_source=chatgpt.com [retrieved on 2025-10-28] * page 7 * | 15 | |
| A | US 2013/261415 A1 (ASHE JEFFREY MICHAEL [US] ET AL) 3 October 2013 (2013-10-03) * paragraphs [0065] - [0068] * | 9 | |

**TECHNICAL FIELDS SEARCHED (IPC)**

A61B

The present search report has been drawn up for all claims

| Place of search | Date of completion of the search | Examiner |
|---|---|---|
| Berlin | 7 November 2025 | Pohjamo, Terhi |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 19 2243

This annex lists the patent family members relating to the patent documents cited in the above-mentioned European search report.
The members are as contained in the European Patent Office EDP file on
The European Patent Office is in no way liable for these particulars which are merely given for the purpose of information.

07-11-2025

| Patent document cited in search report | | Publication date | Patent family member(s) | | Publication date |
|---|---|---|---|---|---|
| US 8761850 | B2 | 24-06-2014 | US | 2010094107 A1 | 15-04-2010 |
| | | | US | 2013211264 A1 | 15-08-2013 |
| | | | US | 2014296720 A1 | 02-10-2014 |
| US 2013035562 | A1 | 07-02-2013 | EP | 2448466 A1 | 09-05-2012 |
| | | | US | 2010331638 A1 | 30-12-2010 |
| | | | US | 2013035562 A1 | 07-02-2013 |
| | | | WO | 2011008382 A1 | 20-01-2011 |
| EP 0352923 | A1 | 31-01-1990 | EP | 0352923 A1 | 31-01-1990 |
| | | | JP | H0274862 A | 14-03-1990 |
| US 2013261415 | A1 | 03-10-2013 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63678217